# EUROPEAN PATENT APPLICATION

(11) **EP 0 943 682 A1**
(43) Date of publication of application: **22.09.1999**
(21) Application number: 98103334.3
(22) Date of filing: 26.02.1998
(51) Int. Cl.: C12N 15/12, C07K 14/47

(54) **Tumor suppressor gene del-27 and the protein coded thereby, methods of production and use thereof**

(71) Applicant: Roche Diagnostics GmbH, 68305 Mannheim (DE)
(72) Inventor: The designation of the inventor has not yet been filed

(57) **Abstract**

The invention comprises an isolated nucleic acid molecule encoding a tumor suppressor gene, designated del-27, as well as nucleic acids which hybridize to this molecule under stringent conditions, and proteins encoded by these nucleic acids. The tumor suppressor gene was identified in the del-27 locus on the long arm of human chromosome 5, an area identified by frequent loss of heterozygosity in tumor cells.

## Description

The present invention relates to the new tumor suppressor gene del-27, the protein coded thereby, and their use for diagnostics and therapeutics, especially in the field of cancer. In particular, the invention relates to the diagnosis of the lack of tumor suppressor gene del-27 in mammalian, especially in tumor, cells and gene therapy methods to restore del-27 and its functions in mammalian cells, especially in tumor cells.

Tumor suppressor genes are typically thought of as genes whose expression is reduced or lost in cancer cells (Knudson, Proc. Natl. Acad. Sci. USA 19 (1993) 10914-10921). The lack of expression results from mutations in the genes encoding their proteins. Since these proteins are believed to suppress cell growth and thereby act as negative growth regulators, loss of their expression in tumor cells leads to the increased cell proliferation observed and contributes to malignant transformation. As negative growth regulators, tumor suppressor gene products are likely to have also normal functions critical to the development of differentiated tissues. In this respect, tumor suppressor genes may have an important role in the growth arrest necessary for the onset of cellular differentiation as growth regulation is a normal feature of development and differentiation. An overview of tumor suppressor genes is given by, e.g., Gutmann, D.H., Int. J. Dev. Biol. 39 (1995) 895-907.

Inactivation of tumor suppressor genes appears to be a predominant genetic event in the genesis and progression of many tumors. In normal cells, these genes are thought to be involved in the regulation of cell proliferation and differentiation (Fearon, E., and Vogelstein, B., Cell 61 (1990) 759-767). Inactivating mutations and deletions of tumor suppressor genes may therefore release normal growth constraints and may result in the development or progression of tumor cells. A genomic region that contains a putative tumor suppressor gene can be identified by frequent loss of heterozygosity (LOH) of the normal allele with the remaining allele beeing presumably non-functional in the tumor cells (Fearon, E., and Vogelstein, B., Cell 61 (1990) 759-767).

Recently, frequent LOH at the del-27 locus on the long arm of chromosome 5 in human lung carcinomas was demonstrated (Wieland, I., and Böhm, M., Cancer Res. 54 (1994) 1772-1774; Wieland, I., et al., Oncogene 12 (1996) 97-102). Furthermore, LOH at the del-27 locus correlated with tumor progression in transitional cell carcinoma (Böhm, M., Int. J. Cancer 72 (1997) 1-5). del-27 was isolated by genomic difference cloning and is homozygously deleted in a lung carcinoma cell line supporting its close linkage to a novel putative tumor suppressor gene (Wieland, I., Proc. Natl. Acad. Sci. USA 87 (1992) 2720-2724). However, no cDNA coding for a tumor suppressor gene was described therein.

It is an object of the invention to provide a novel tumor suppressor gene from the del-27 locus. The invention therefore comprises an isolated nucleic acid molecule (del-27)
a) with the nucleic acid sequence SEQ ID NO:1 or
b) which hybridizes under stringent conditions with all nucleic acid fragments 477-819, 820-3069 and 3070-3137 or the complementary nucleic acids
   and which is capable of suppressing cell proliferation.

The invention further comprises a recombinant polypeptide which suppresses cell proliferation and
a) is coded by the DNA sequence shown in SEQ ID NO:1,
b) is coded by DNA sequences which hybridize under stringent conditions with all of the nucleic acid fragments 477-819, 820-3069 and 3070-3137,
c) is coded by DNA sequences which, if there was no degeneracy of the genetic code, would hybridize under stringent conditions with the sequences defined in a) and b).

The polypeptide can be defined by its DNA sequence and by the amino acid sequence derived therefrom. The del-27 polypeptide can occur in natural allelic variations which differ from individual to individual. Such variations of the amino acids are usually amino acid substitutions. However, they may also be deletions, insertions or additions of amino acids to the total sequence. The del-27 protein according to the invention - depending, both in respect of the extent and type, on the cell and cell type in which it is expressed- can be in glycosylated or non-glycosylated form. Polypeptides with tumor-suppressive activity can easily be identified by a tumor growth inhibition assay using carcinoma cells expressing said polypeptides and measuring the proliferation capacity and apoptosis in relation to carcinoma cells not expressing said polypeptides.

"Polypeptide with del-27 activity or del-27" means also proteins with minor amino acid variations but with substantially the same del-27 activity. Substantially the same means that the activities are of the same biological properties and the polypeptides show preferably at least 75% homology in amino acid sequence. More preferably, the amino acid sequences are at least 90% identical.

The term "nucleic acid molecule" denotes a polynucleotide which can be, for example, a DNA, RNA, or derivatized active DNA or RNA. DNA and/or RNA molecules are preferred, however.

The term "hybridize under stringent conditions" means that two nucleic acid fragments are capable of hybridization to one another under standard hybridization conditions described in Sambrook et al., "Expression of cloned genes in E. coli" in Molecular Cloning: A laboratory manual (1989) Cold Spring Harbor Laboratory Press, New York, USA, 9.47 - 9.62 and 11.45 - 11.61.

More specifically, "stringent conditions" as used herein refer to hybridization in 6.0 x SSC at about 45°C, followed by a wash of 2.0 x SSC at 50°C. For selection of the stringency the salt concentration in the wash step can be selected, for example from about 2.0 x SSC at 50°C, for low stringency, to about 0.2 x SSC at 50°C, for high stringency. In addition, the temperature in the wash step can be increased from low stringency conditions at room temperatures, about 22°C, to high stringency conditions at about 65°C.

The term "isolated" as used throughout this application refers to a nucleic acid or polypeptide having an del-27 activity and is substantially free of cellular material or culture medium, when produced by recombinant DNA techniques, or chemical precursors or other chemicals, when synthesized chemically. An isolated nucleic acid is preferably free of sequences which naturally flank the nucleic acid (i.e. sequences located at the 5' and the 3' ends of the nucleic acid) in the organism from which the nucleic acid is derived.

del-27 can be purified after recombinant production by affinity chromatography using known protein purification techniques, including immunoprecipitation, gel filtration, ion exchange chromatography, chromatofocussing, isoelectric focussing, selective precipitation, electrophoresis, and the like.

The polypeptides according to the invention can also be produced by recombinant means, or synthetically. Non-glycosylated del-27 polypeptide is obtained when it is produced recombinantly in prokaryotes. With the aid of the nucleic acid sequences provided by the invention it is possible to search for the del-27 gene or its variants in genomes of any desired cells (e.g. apart from human cells, also in cells of other mammals), to identify these and to isolate the desired gene coding for the del-27 protein. Such processes and suitable hybridization conditions are known to a person skilled in the art and are described, for example, by Sambrook, J., et al., "Expression of cloned genes in E. coli" in Molecular Cloning: A laboratory manual (1989) Cold Spring Harbor Laboratory Press, New York, USA, and B.D. Hames, S.G. Higgins, Nucleic acid hybridisation - a practical approach (1985) IRL Press, Oxford, England. In this case the standard protocols described in these publications are usually used for the experiments.

The use of recombinant DNA technology enables the production of numerous active del-27 derivatives. Such derivatives can, for example, be modified in individual or several amino acids by substitution, deletion or addition. The derivatization can, for example, be carried out by means of site directed mutagenesis. Such variations can be easily carried out by a person skilled in the art (J. Sambrook, B.D. Hames, loc. cit.). It merely has to be ensured by means of the below-mentioned tumor cell growth inhibition assay that the characteristic properties of del-27 are preserved. The invention therefore in addition concerns an del-27 polypeptide which is a product of a prokaryotic or eukaryotic expression of an exogenous DNA.

With the aid of such nucleic acids coding for an del-27 protein, the protein according to the invention can be obtained in a reproducible manner and in large amounts. For expression in prokaryotic or eukaryotic organisms, such as prokaryotic host cells or eukaryotic host cells, the nucleic acid is integrated into suitable expression vectors, according to methods familiar to a person skilled in the art. Such an expression vector preferably contains a regulatable/inducible promoter. These recombinant vectors are then introduced for the expression into suitable host cells such as, e.g., E. coli as a prokaryotic host cell or Saccharomyces cerevisiae, Terato carcinoma cell line PA-1 sc 9117 (Büttner et al., Mol. Cell. Biol. 11 (1991) 3573-3583), insect cells, CHO or COS cells as eukaryotic host cells and the transformed or transduced host cells are cultured under conditions which allow an expression of the heterologous gene. The isolation of the protein can be carried out according to known methods from the host cell or from the culture supernatant of the host cell. Such methods are described for example by Ausubel I., Frederick M., Current Protocols in Mol. Biol. (1992), John Wiley and Sons, New York. Also in vitro reactivation of the protein may be necessary if it is not found in soluble form in the cell culture.

The invention further comprises recombinant expression vectors which are suitable for the expression of del-27, recombinant host cells transfected with such expression vectors, as well as a process for the recombinant production of a protein which is coded by a tumor suppressor gene del-27.

The invention further comprises a method for detecting a nucleic acid molecule of tumor suppressor gene del-27, comprising incubating a sample (e.g. body fluids such as blood, cell lysates) with the isolated nucleic acid molecule according to the invention and determining hybridization under stringent conditions of said isolated nucleic acid molecule to a target nucleic acid molecule as a determination of presence of a nucleic acid molecule which is the del-27 tumor suppressor gene.

The invention further comprises a method for producing a protein which is capable of suppressing cell proliferation by expressing an exogenous DNA in prokaryotic or eukaryotic host cells and isolation of the desired protein, wherein the DNA
a) is coded by the DNA sequence shown in SEQ ID NO:1,
b) is coded by DNA sequences which hybridize under stringent conditions with all of the nucleic acid fragments 477-819, 820-3069 and 3070-3137,
c) is coded by DNA sequences which, if there was no degeneracy of the genetic code, would hybridize under stringent conditions with the sequences defined in a) and b).

The invention further comprises an isolated protein according to the invention which is encoded by a nucleic acid molecule having the nucleotide sequence set forth in SEQ ID NO:1.

The present invention relates to the cloning and characterization of a tumor suppressor gene which is especially characterized as a tumor progression gene. The functional loss of the tumor suppressor gene according to the invention (del-27) releases contact inhibition and anchorage dependence in tumor cells. Therefore the loss of del-27 gene correlates with a more aggressive behavior of the tumor cells and also the potential of the formation of metastasis.

According to the invention del-27 and its expression products can be used to inhibit tumor growth, preferably of sporadic tumors (in particular, lung and bladder carcinomas) in vivo, preferably by somatic gene therapy.

The following examples, references and sequence listing are provided to aid the understanding of the present invention, the true scope of which is set forth in the appended claims. It is understood that modifications can be made in the procedures set forth without departing from the spirit of the invention.

### Materials and methods

### Screening and analysis of phage clones:

Phage pools containing 10⁵ plaque forming units produced from normal human genomic libraries (Clontech, Cat.# HL1067J; Stratagene, Cat.# 946205) and human fetal and placental cDNA libraires (Clontech, Cat.# HL1065a and # HL1075b) were screened by the polymerase chain reaction (PCR). PCR conditions were as described (Wieland, I., and Böhm, M., Cancer Res. 54 (1994) 1772-1774). For plaque screening filter lifts were hybridized with ³²P-labeled probes and washed under standard conditions (Sambrook, J., et al., Molecular Cloning: A Laboratory Manual, Cold Spring Harbor, N.Y. Cold Spring Harbor Laboratory, 1989). Postive phage were rescreened by PCR and plaque lift hybridization. Single positive plaques were isolated and phage DNA was prepared using a kit (Qiagen, Cat.# 12523). Inserts were analysed by restriction enzyme cleavage and Southern blot hybridization. For genomic clones, subclones of the phage inserts were produced by cleavage with SacI (Boehringer Mannheim) and cloning (Sambrook, J., et al., Molecular Cloning: A Laboratory Manual, Cold Spring Harbor, N.Y. Cold Spring Harbor Laboratory, 1989) into the SacI site of plasmid vector pT7T3 (Stratagene). Selection of single-copy fragments was based on agarose gel electrophoresis of restriction enzyme cleaved inserts and counter-selection of repetitive sequences using total genomic DNA as a probe in Southern blot hybridizations (Seltmann, M., et al., Cytogenet. Cell Genet. 67 (1994) 46-51). Genomic Southern blot hybridizations were performed as described (Wieland, I., and Böhm, M., Cancer Res, 54/1994) 1772-1774; Wieland, I., et al., Proc. Natl. Acad. Sci. USA 87 (1992) 2720-2724).

### DNA sequencing:

Both strands of overlapping cDNA clones were sequenced commerically (Eurogentec, Seraing, Bel.). DNA sequences were analysed using DNAsis, sequence analysis software (Hitachi).

### Cell lines and tumor specimens:

Human lung cancer cell lines were obtained and cultivated as described (Wieland, I., and Böhm, M., Cancer Res, 54/1994) 1772-1774; Wieland, I., et al., Proc. Natl. Acad. Sci. USA 87 (1992) 2720-2724).

### Results

### Analysis of the del-27 genomic region:

For analysis of the region flanking the del-27 sequence normal human genomic phage libraries were screened by PCR for the presence of a 356bp PCR product of the del-27 sequence (Wieland, I., and Böhm, M., Cancer Res, 54/1994) 1772-1774). Two overlapping phage clones covering 21kb of the del-27 region were isolated and subcloned into a plasmid vector. Single-copy subclones from the del-27 region were used as hybridization probes on genomic Southern blots. Probe p17E that contains an internal HindII site hybridized to a 4.3kb and a second larger HindII fragment. In human lung carcinoma cell line SK-LC-17, however, the 4.3kb HindII fragment was missing while a smaller fragment of 1.3 kb was apparent. Thus, subclone p17E identified a homozygous deletion of 3kb in this cell line. This homozygous deletion includes the previously isolated del-27 sequence. A PstI site was also lost resulting in a rearranged PstI restriction fragment. Only 3.5kb apart from this deletion a second homozygous deletion of 1.4kb was identified in the same cell line using subclone p13B as a probe. Further homozygous deletions or rearrangements within the 21kb region were not observed by Southern blotting experiments. This demonstrates that homozygous deletions accompanied by genomic rearrangements occurred within 10kb of the del-27 region in human lung carcinoma cell line SK-LC-17. Probe p13B produced, in addition to the strong hybridizing signal from the del-27 region on chromosome 5, weaker signals on genomic Southern blots. To determine whether probe p13B cross-hybridized with another chromosomal region in the human genome a somatic cell hybrid panel was analysed (Wieland, I., et al., Proc. Natl. Acad. Sci. USA 87 (1992) 2720-2724). Cross-hybridizing signals were detected in somatic cell hybrids containing human chromosome 13. This indicates that a sequence homologous to the p13B sequence from the del-27 region exists on human chromosome 13. To investigate whether subclones p17E and p13B detect potential exon sequences they were analysed for cross-species conservation. Both subclones hybridized to genomic fragments from several species. In particular, subclone p13B appeared to be highly conserved because it hybridized to all mammalian DNAs tested (man, African green monkey, mouse, rat, hamster, dog, cow) and also to chicken. This suggested that exon sequences of a gene exist in the del-27 region.

### Isolation of the del-27 cDNA:

Human cDNA libraries were screened by PCR using primers deduced from the highly conserved clone p13B. Several overlapping clones were isolated and sequenced. The aligned cDNA of 3665bp contains an open reading frame of 2661bp (from nucleotide postion 477 to 3137). This corresponds to an 887 amino acid sequence. Comparison of the del-27 cDNA with known cDNAs from GeneBank identified a region of homology (with 90% identity) from nucleotide position 820 to 3069 to a mouse cDNA (GenBank accession number U57368, MMU57368) encoding a putative transmembrane protein involved in contact inhibition and anchorage dependent growth.

These results suggest that functional loss of the del-27 gene and/or related genes releases contact inhibition and anchorage dependence in tumor cells. This is supported by the observation that loss of the del-27 gene correlates with a more aggressive behavior of the tumor cells in bladder carcinoma (Böhm, M., et al., Int. J. Cancer 72 (1997) 1-5).

### Functional test of the del-27 cDNA as a tumor suppressor gene:

The tumor suppressive activity of the del-27 cDNA can be tested by introduction of either the entire coding region or parts of the coding region of the del-27 cDNA into carcinoma cells:

The entire coding region or in frame parts of the coding region are cloned into the tetracycline-inducible expression vector pUHD10-3 (Gossen, M., and Bujard, H., Proc. Natl. Acad. Sci. USA 89 (1992) 5547-5551). Together with a selection marker the different constructs are then electroporated into carcinoma cells containing the rtTa-gene on plasmid pUHD172-1neo (Gossen, M., et al., Science 268 (1995) 1766-1769). In selected cell clones expression of the del-27 cDNA is induced by treatment of the cells with tetracycline or doxycycline. In vitro, tumor suppressive activity of the del-27 cDNA constructs are determined by measuring the proliferative capacity and apoptosis of induced cells compared to non-induced cells. The generated carcinoma cells are further investigated in an *in vivo* mouse model. Cells are injected into immune-deficient mice. Expression of the del-27 cDNA constructs in the carcinoma cells is induced by tetracycline when small tumor nodules are apparent. Tumor suppression (tumor growth inhibition, tumor regression) is monitored in the induced and non-induced state.

### Future application:

In combination with retro- or adenoviral vectors the observed tumor suppressive activity of the del-27 cDNA or products of it can be used to inhibt tumor growth of sporadic tumors (in particular in carcinomas such as lung and bladder carcinomas) in vivo by somatic gene therapy.

### List of References

Ausubel I., Frederick M., Current Protocols in Mol. Biol. (1992), John Wiley and Sons, New York
Böhm, M., et al., Int. J. Cancer 72 (1997) 1-5
Büttner et al., Mol. Cell. Biol. 11 (1991) 3573-3583
Fearon, E., and Vogelstein, B., Cell 61 (1990) 759-767
Gossen, M., and Bujard, H., Proc. Natl. Acad. Sci. USA 89 (1992) 5547-5551
Gossen, M., et al., Science 268 (1995) 1766-1769
Gutmann, D.H., Int. J. Deph. Biol. 39 (1995) 895-907
Hames, B.D., Higgins, S.G., Nucleic acid hybridisation - a practical approach (1985) IRL Press, Oxford, England
Knudson, Proc. Natl. Acad. Sci. USA 19 (1993) 10914-10921
Sambrook et al., "Expression of cloned genes in E. coli" in Molecular Cloning: A laboratory manual (1989) Cold Spring Harbor Laboratory Press, New York, USA, 9.47 - 9.62 and 11.45 - 11.61
Seltmann, M., et al., Cytogenet. Cell Genet. 67 (1994) 46-51
Wieland, I., and Böhm, M, Cancer Res. 54 (1994) 1772-1774
Wieland, I., et al., Oncogene 12 (1996) 97-102
Wieland, I., et al., Proc. Natl. Acad. Sci. USA 87 (1992) 2720-2724

## Claims

1. An isolated nucleic acid molecule (del-27)
a) with the nucleic acid sequence SEQ ID NO:1 or
b) which hybridizes under stringent conditions with all nucleic acid fragments 477-819, 820-3069 and 3070-3137 or the complementary nucleic acids
and which is capable of suppressing cell proliferation.

2. A recombinant expression vector which is suitable for the expression of a nucleic acid molecule as claimed in claim 1.

3. A recombinant polypeptide which suppresses cell proliferation and
a) is coded by the DNA sequence shown in SEQ ID NO:1,
b) is coded by DNA sequences which hybridize under stringent conditions with all of the nucleic acid fragments 477-819, 820-3069 and 3070-3137,
c) is coded by DNA sequences which, if there was no degeneracy of the genetic code, would hybridize under stringent conditions with the sequences defined in a) and b).

4. A method for the production of a protein which has tumor suppressive activity, by expressing an exogenous DNA in prokaryotic or eukaryotic host cells and isolation of the desired protein, wherein the DNA
a) is coded by the DNA sequence shown in SEQ ID NO:1,
b) is coded by DNA sequences which hybridize under stringent conditions with all of the nucleic acid fragments 477-819, 820-3069 and 3070-3137,
c) is coded by DNA sequences which, if there was no degeneracy of the genetic code, would hybridize under stringent conditions with the sequences defined in a) and b).


SEQUENCE LISTING

(1) GENERAL INFORMATION:

(i) APPLICANT:
(A) NAME: BOEHRINGER MANNHEIM GMBH
(B) STREET: Sandhofer Str. 116
(C) CITY: Mannheim
(E) COUNTRY: Germany
(F) POSTAL CODE (ZIP): D-68305
(G) TELEPHONE: 08856/60-3446
(H) TELEFAX: 08856/60-3451

(ii) TITLE OF INVENTION: New tumor suppressor gene and the protein
coded thereby, methods of production and use thereof

(iii) NUMBER OF SEQUENCES: 2

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30B (EPO)

(2) INFORMATION FOR SEQ ID NO: 1:

(i) SEQUENCE CHARACTERISTICS:
(A) LENGTH: 3691 base pairs
(B) TYPE: nucleic acid
(C) STRANDEDNESS: double
(D) TOPOLOGY: linear

(ii) MOLECULE TYPE: cDNA

(ix) FEATURE:
(A) NAME/KEY: CDS
(B) LOCATION:477..3137

(xi) SEQUENCE DESCRIPTION: SEQ ID NO: 1:

GGGCCGGGGT GCGGAGGGAG TCCCTGAAAC TTCTCCCAAC AGCCCCAGGG CCAATCTTCC 60

CTCAGCCCAC TCCCCTCTTC GCCAGCTCCC AGGTCCCCAC TCCTGTACCG GGGGTGGGGT 120

GGGGAGCCAG GCCCGTCTCC CGCTGGGACA CACACAGGGG CCGGGGAGCG GGGACGGGAC 180

CCCCGAGGCG GGGGGGACGA ACCGACAGAC AGACGGCTGG GCGCCCGCCC CAGCGGGCAG 240

GCAGGCAGGA GGAGGCGGAG GCGGGGGTAC GACGGGGAGC ACTGGGTCTG GGGAGTTTCC 300

TCTCAACTAT CGGGGGAGAA ACTCCCCGCA GCCGGAGGAA AGACCCAGAC AGTGTTTTCC 360

TCCCGGGGGC CGTGCTCCCC CGCCCCGCGT AGCGGCGGTC GCCGCCACCG GCGCCTCCAC 420

CTCTACCATC TCCTCTTTCT CCACCACCTC GGGCCCCGGT GTCCCCGGCC AGCACT 476

ATG CCC ATC TTA CTG TTC CTG ATA GAC ACG TCT GCC TCT ATG AAC CAG 524
Met Pro Ile Leu Leu Phe Leu Ile Asp Thr Ser Ala Ser Met Asn Gln
1               5                   10                  15

CGC AGC CAT CTG GGC ACC ACC TAC CTG GAC ACG GCC AAA GGC GCG GTA 572
Arg Ser His Leu Gly Thr Thr Tyr Leu Asp Thr Ala Lys Gly Ala Val
            20                  25                  30

GAG ACC TTC ATG AAG CTC CGT GCC CGG GAC CCT GCC AGC AGA GGA GAC 620
Glu Thr Phe Met Lys Leu Arg Ala Arg Asp Pro Ala Ser Arg Gly Asp
        35                  40                  45

AGG TAT ATG CTG GTC ACT TTC GAA GAG CCG CCC TAT GCT ATC AAG GCT 668
Arg Tyr Met Leu Val Thr Phe Glu Glu Pro Pro Tyr Ala Ile Lys Ala
    50                  55                  60

GGA TGG AAA GAA AAC CAT GCA ACG TTT ATG AAT GAA TTG AAA AAC CTT 716
Gly Trp Lys Glu Asn His Ala Thr Phe Met Asn Glu Leu Lys Asn Leu
65                  70                  75                  80

CAG GCT GAA GGA CTT ACG ACT CTT GGC CAA TCC CTA AGG ACA GCT TTT 764
Gln Ala Glu Gly Leu Thr Thr Leu Gly Gln Ser Leu Arg Thr Ala Phe
                85                  90                  95

GAT TTA TTA AAT TTA AAT AGA TTA GTA ACT GGC ATA GAC AAC TAT GGG 812
Asp Leu Leu Asn Leu Asn Arg Leu Val Thr Gly Ile Asp Asn Tyr Gly
            100                 105                 110

CAG GGA AGA AAC CCT TTT TTC TTG GAG CCA GCA ATA ATT ATC ACA ATT 860
Gln Gly Arg Asn Pro Phe Phe Leu Glu Pro Ala Ile Ile Ile Thr Ile
        115                 120                 125

ACT GAT GGG AGC AAG TTG ACT ACC ACC AGT GGA GTC CAG GAT GAG CTT 908
Thr Asp Gly Ser Lys Leu Thr Thr Thr Ser Gly Val Gln Asp Glu Leu
    130                 135                 140

CAT TTA CCT CTT AAT TCT CCT TTG CCT GGA AGT GAA TTG ACC AAG GAA 956
His Leu Pro Leu Asn Ser Pro Leu Pro Gly Ser Glu Leu Thr Lys Glu
145                 150                 155                 160

CCT TTT CGT TGG GAT CAG AGA CTC TTT GCA TTA GTG TTG CGG TTG CCT 1004
Pro Phe Arg Trp Asp Gln Arg Leu Phe Ala Leu Val Leu Arg Leu Pro
                165                 170                 175

GGC ACC ATG TCA GTA GAA TCA GAA CAG TTG ACA GGT GTG CCT TTA GAT 1052
Gly Thr Met Ser Val Glu Ser Glu Gln Leu Thr Gly Val Pro Leu Asp
            180                 185                 190

GAC TCT GCA ATC ACA CCA ATG TGT GAA GTG ACA GGC GGC CGT TCA TAT 1100
Asp Ser Ala Ile Thr Pro Met Cys Glu Val Thr Gly Gly Arg Ser Tyr
        195                 200                 205

TCT GTG TGT TCT CCA AGA ATG CTT AAT CAG TGT CTG GAG TCC TTG GTG 1148
Ser Val Cys Ser Pro Arg Met Leu Asn Gln Cys Leu Glu Ser Leu Val
    210                 215                 220

CAG AAA GTA CAA AGT GGG GTG GTA ATA AAC TTT GAA AAA GCA GGA CCA 1196
Gln Lys Val Gln Ser Gly Val Val Ile Asn Phe Glu Lys Ala Gly Pro
225                 230                 235                 240

GAT CCT TCC CCT GTA GAA GAT GGG CAG CCA GAT ATA TCA AGG CCT TTT 1244
Asp Pro Ser Pro Val Glu Asp Gly Gln Pro Asp Ile Ser Arg Pro Phe
                245                 250                 255

GGA TCT CAG CCT TGG CAT AGC TGT CAC AAA CTC ATA TAT GTC AGA CCA 1292
Gly Ser Gln Pro Trp His Ser Cys His Lys Leu Ile Tyr Val Arg Pro
            260                 265                 270

AAT CCT AAA ACT GGG GTT CCT ATA GGT CAT TGG CCT GTT CCA GAG TCT 1340
Asn Pro Lys Thr Gly Val Pro Ile Gly His Trp Pro Val Pro Glu Ser
        275                 280                 285

TTT TGG CCA GAT CAA AAT TCG CCA ACA CTA CCA CCT CGT ACA TCT CAT 1388
Phe Trp Pro Asp Gln Asn Ser Pro Thr Leu Pro Pro Arg Thr Ser His
    290                 295                 300

CCT GTA GTG AAG TTT TCC TGT ACA GAC TGT GAA CCA ATG GTT ATT GAT 1436
Pro Val Val Lys Phe Ser Cys Thr Asp Cys Glu Pro Met Val Ile Asp
305                 310                 315                 320

AAA CTT CCT TTT GAC AAA TAT GAG TTG GAA CCT TCA CCA CTG ACT CAA 1484
Lys Leu Pro Phe Asp Lys Tyr Glu Leu Glu Pro Ser Pro Leu Thr Gln
                325                 330                 335

TTT ATC CTG GAA AGG AAA TCT CCT CAA ACA TGT TGG CAG GTG TAC GTG 1532
Phe Ile Leu Glu Arg Lys Ser Pro Gln Thr Cys Trp Gln Val Tyr Val
            340                 345                 350

AGC AAT AGT GCA AAA TAC AGT GAA CTT GGT CAT CCT TTT GGT TAC TTG 1580
Ser Asn Ser Ala Lys Tyr Ser Glu Leu Gly His Pro Phe Gly Tyr Leu
        355                 360                 365

AAA GCC AGT ACA GCA CTG AAC TGT GTC AAC TTA TTT GTG ATG CCT TAC 1628
Lys Ala Ser Thr Ala Leu Asn Cys Val Asn Leu Phe Val Met Pro Tyr
    370                 375                 380

AAT TAT CCA GTC CTT CTT CCC CTC TTA GAT GAC TTG TTT AAA GTG CAT 1676
Asn Tyr Pro Val Leu Leu Pro Leu Leu Asp Asp Leu Phe Lys Val His
385                 390                 395                 400

AAA GCA AAA CCA ACA TTG AAG TGG AGA CAG TCA TTT GAA AGT TAT TTG 1724
Lys Ala Lys Pro Thr Leu Lys Trp Arg Gln Ser Phe Glu Ser Tyr Leu
                405                 410                 415

AAG ACA ATG CCT CCC TAC TAT CTT GGG CCC TTG AAG AAA GCT GTT AGG 1772
Lys Thr Met Pro Pro Tyr Tyr Leu Gly Pro Leu Lys Lys Ala Val Arg
            420                 425                 430

ATG ATG GGA GCA CCT AAC CTA ATA GCA GAC AGT ATG GAA TAT GGA CTT 1820
Met Met Gly Ala Pro Asn Leu Ile Ala Asp Ser Met Glu Tyr Gly Leu
        435                 440                 445

AGT TAC AGT GTC ATT TCA TAC CTC AAA AAA CTG AGT CAA CAG GCC AAA 1868
Ser Tyr Ser Val Ile Ser Tyr Leu Lys Lys Leu Ser Gln Gln Ala Lys
    450                 455                 460

ATA GAA TCT GAT CGA GTC ATT GGA TCT GTA GGC AAA AAA GTA GTA CAG 1916
Ile Glu Ser Asp Arg Val Ile Gly Ser Val Gly Lys Lys Val Val Gln
465                 470                 475                 480

GAG ACT GGA ATA AAA GTC CGG AGC CGA TCA CAT GGT TTA TCA ATG GCA 1964
Glu Thr Gly Ile Lys Val Arg Ser Arg Ser His Gly Leu Ser Met Ala
                485                 490                 495

TAT AGG AAA GAT TTT CAA CAA CTC CTC CAG GGA ATT TCA GAG GAT GTC 2012
Tyr Arg Lys Asp Phe Gln Gln Leu Leu Gln Gly Ile Ser Glu Asp Val
            500                 505                 510

CCT CAC AGA CTG CTA GAC CTT AAT ATG AAG GAA TAC ACT GGG TTC CAA 2060
Pro His Arg Leu Leu Asp Leu Asn Met Lys Glu Tyr Thr Gly Phe Gln
        515                 520                 525

GTT GCT TTG CTG AAT AAG GAT TTG AAG CCA CAG ACA TTT AGA AAT GCT 2108
Val Ala Leu Leu Asn Lys Asp Leu Lys Pro Gln Thr Phe Arg Asn Ala
    530                 535                 540

TAT GAC ATA CCA AGA CGA AAT CTT TTG GAT CAC TTA ACA AGA ATG AGA 2156
Tyr Asp Ile Pro Arg Arg Asn Leu Leu Asp His Leu Thr Arg Met Arg
545                 550                 555                 560

TCT AAT CTT TTG AAG AGC ACT CGC AGA TTT CTG AAA GGA CAG GAC GAA 2204
Ser Asn Leu Leu Lys Ser Thr Arg Arg Phe Leu Lys Gly Gln Asp Glu
                565                 570                 575

GAT CAA GTG CAC AGT GTT CCT ATA GCA CAA ATG GGG AAC TAC CAG GAA 2252
Asp Gln Val His Ser Val Pro Ile Ala Gln Met Gly Asn Tyr Gln Glu
            580                 585                 590

TAC CTC AAG CAA GTA CCT TCT CCA CTA AGA GAA CTT GAT CCT GAT CAG 2300
Tyr Leu Lys Gln Val Pro Ser Pro Leu Arg Glu Leu Asp Pro Asp Gln
        595                 600                 605

CCA CGA AGG TTG CAT ACA TTT GGC AAC CCC TTT AAG CTG GAT AAG AAG 2348
Pro Arg Arg Leu His Thr Phe Gly Asn Pro Phe Lys Leu Asp Lys Lys
    610                 615                 620

GGT ATG ATG ATA GAT GAA GCA GAT GAA TTT GTG GCT GGA CCT CAA AAT 2396
Gly Met Met Ile Asp Glu Ala Asp Glu Phe Val Ala Gly Pro Gln Asn
625                 630                 635                 640

AAA CAT AAA CGA CCC GGA GAA CCA AAT ATG CAA GGG ATC CCT AAA AGA 2444
Lys His Lys Arg Pro Gly Glu Pro Asn Met Gln Gly Ile Pro Lys Arg
                645                 650                 655

CGT CGG TGT ATG TCT CCA CTA CTA AGA GGC AGA CAG CAG AAT CCT GTT 2492
Arg Arg Cys Met Ser Pro Leu Leu Arg Gly Arg Gln Gln Asn Pro Val
            660                 665                 670

GTA AAC AAT CAT ATT GGG GGA AAA GGA CCA CCT GCA CCT ACA ACT CAA 2540
Val Asn Asn His Ile Gly Gly Lys Gly Pro Pro Ala Pro Thr Thr Gln
        675                 680                 685

GCA CAG CCA GAT CTT ATT AAA CCT CTT CCT CTT CAT AAA ATT TCA GAA 2588
Ala Gln Pro Asp Leu Ile Lys Pro Leu Pro Leu His Lys Ile Ser Glu
    690                 695                 700

ACC ACT AAT GAT TCG ATA ATA CAT GAT GTG GTT GAA AAT CAT GTT GCA 2636
Thr Thr Asn Asp Ser Ile Ile His Asp Val Val Glu Asn His Val Ala
705                 710                 715                 720

GAC CAA CTT TCA TCA GAC ATT ACA CCA AAT GCT ATG GAT ACG GAA TTT 2684
Asp Gln Leu Ser Ser Asp Ile Thr Pro Asn Ala Met Asp Thr Glu Phe
                725                 730                 735

TCA GCA TCT TCT CCA GCC AGT TTA CTG GAA CGG CCA ACC AAT CAT ATG 2732
Ser Ala Ser Ser Pro Ala Ser Leu Leu Glu Arg Pro Thr Asn His Met
            740                 745                 750

GAG GCT CTT GGT CAT GAC CAT TTA GGA ACC AAT GAC CTC ACT GTT GGT 2780
Glu Ala Leu Gly His Asp His Leu Gly Thr Asn Asp Leu Thr Val Gly
        755                 760                 765

GGA TTT TTA GAA AAT CAT GAG GAG CCA AGA GAT AAA GAA CAA TGT GCT 2828
Gly Phe Leu Glu Asn His Glu Glu Pro Arg Asp Lys Glu Gln Cys Ala
    770                 775                 780

GAA GAG AAC ATA CCA GCA TCT TCA CTC AAC AAA GGA AAG AAA TTG ATG 2876
Glu Glu Asn Ile Pro Ala Ser Ser Leu Asn Lys Gly Lys Lys Leu Met
785                 790                 795                 800

CAT TGC AGA AGC CAT GAA GAG GTC AAT ACT GAA CTA AAA GCA CAA ATA 2924
His Cys Arg Ser His Glu Glu Val Asn Thr Glu Leu Lys Ala Gln Ile
                805                 810                 815

ATG AAA GAG ATC CGA AAG CCA GGA AGA AAA TAT GAA AGA ATC TTC ACT 2972
Met Lys Glu Ile Arg Lys Pro Gly Arg Lys Tyr Glu Arg Ile Phe Thr
            820                 825                 830

TTA CTG AAG CAT GTG CAA GGC AGT TTA CAA ACA AGA CTA ATA TTT TTA 3020
Leu Leu Lys His Val Gln Gly Ser Leu Gln Thr Arg Leu Ile Phe Leu
        835                 840                 845

CAA AAT GTC ATT AAA GAA GCA TCA AGG TTT AAA AAA CGA ATG CTA ATA 3068
Gln Asn Val Ile Lys Glu Ala Ser Arg Phe Lys Lys Arg Met Leu Ile
    850                 855                 860

GAA CAA CTG GAG AAC TTC TTG GAT GAA ATT CAT CGA AGA GCC AAT CAG 3116
Glu Gln Leu Glu Asn Phe Leu Asp Glu Ile His Arg Arg Ala Asn Gln
865                 870                 875                 880

ATC AAC CAT ATT AAT AGC AAT TAAAAGAAAA TAGAATGTGG CCACTTATTT 3167
Ile Asn His Ile Asn Ser Asn
                885

CACTATCTTC TTCAAATACA AAGTAAATAA CAAGACTGTT GTGATCTTGC ATTCATTTTC 3227

TGACATGCAT TGTTGGCTAT TTGAAATACT AAAAGCAAAT CTACAGATCC TTTTTCCATC 3287

ATTTTACAGT GACCTTTTCT TCATTTTGGT TTATTTTTGT AATGTGAAAA GTATCACTCT 3347

AAAAAACATT TTTAATTTAA CAAACTAAAA ATATTCCTCC AAATCTCTTG CTTGTCATTG 3407

ACTCTTGCGT GTCAATTTCC CTCAGGTTCT ATTTTCTTAA ACCAACCTTT AAAATTGTCA 3467

CCTCTGTTAA GGTTGAACTT TGCCAAAAAA AAAAGAAGTT ACTTTGTAAT TTTTGGGGAA 3527

AAAAGCACAT ACATTAAAAC TAGGTAATGT TTTGTATATA CAGTTATTTT GGATATATTA 3587

TTGTAAGTTG TACAAATGTA TTTTGAAGAA TATTTAAGAA AAGCACTTTT GTTATTCCAT 3647

CAATAAATGC TCTATTTTAA AAAAAAAAAA AAAAAAAAAA AAAA 3691

(2) INFORMATION FOR SEQ ID NO: 2:

(i) SEQUENCE CHARACTERISTICS:
(A) LENGTH: 887 amino acids
(B) TYPE: amino acid
(D) TOPOLOGY: linear

(ii) MOLECULE TYPE: protein

(xi) SEQUENCE DESCRIPTION: SEQ ID NO: 2:

Met Pro Ile Leu Leu Phe Leu Ile Asp Thr Ser Ala Ser Met Asn Gln
1               5                   10                  15

Arg Ser His Leu Gly Thr Thr Tyr Leu Asp Thr Ala Lys Gly Ala Val
            20                  25                  30

Glu Thr Phe Met Lys Leu Arg Ala Arg Asp Pro Ala Ser Arg Gly Asp
        35                  40                  45

Arg Tyr Met Leu Val Thr Phe Glu Glu Pro Pro Tyr Ala Ile Lys Ala
    50                  55                  60

Gly Trp Lys Glu Asn His Ala Thr Phe Met Asn Glu Leu Lys Asn Leu
65                  70                  75                  80

Gln Ala Glu Gly Leu Thr Thr Leu Gly Gln Ser Leu Arg Thr Ala Phe
                85                  90                  95

Asp Leu Leu Asn Leu Asn Arg Leu Val Thr Gly Ile Asp Asn Tyr Gly
            100                 105                 110

Gln Gly Arg Asn Pro Phe Phe Leu Glu Pro Ala Ile Ile Ile Thr Ile
        115                 120                 125

Thr Asp Gly Ser Lys Leu Thr Thr Thr Ser Gly Val Gln Asp Glu Leu
    130                 135                 140

His Leu Pro Leu Asn Ser Pro Leu Pro Gly Ser Glu Leu Thr Lys Glu
145                 150                 155                 160

Pro Phe Arg Trp Asp Gln Arg Leu Phe Ala Leu Val Leu Arg Leu Pro
                165                 170                 175

Gly Thr Met Ser Val Glu Ser Glu Gln Leu Thr Gly Val Pro Leu Asp
            180                 185                 190

Asp Ser Ala Ile Thr Pro Met Cys Glu Val Thr Gly Gly Arg Ser Tyr
        195                 200                 205

Ser Val Cys Ser Pro Arg Met Leu Asn Gln Cys Leu Glu Ser Leu Val
    210                 215                 220

Gln Lys Val Gln Ser Gly Val Val Ile Asn Phe Glu Lys Ala Gly Pro
225                 230                 235                 240

Asp Pro Ser Pro Val Glu Asp Gly Gln Pro Asp Ile Ser Arg Pro Phe
                245                 250                 255

Gly Ser Gln Pro Trp His Ser Cys His Lys Leu Ile Tyr Val Arg Pro
            260                 265                 270

Asn Pro Lys Thr Gly Val Pro Ile Gly His Trp Pro Val Pro Glu Ser
        275                 280                 285

Phe Trp Pro Asp Gln Asn Ser Pro Thr Leu Pro Pro Arg Thr Ser His
    290                 295                 300

Pro Val Val Lys Phe Ser Cys Thr Asp Cys Glu Pro Met Val Ile Asp
305                 310                 315                 320

Lys Leu Pro Phe Asp Lys Tyr Glu Leu Glu Pro Ser Pro Leu Thr Gln
                325                 330                 335

Phe Ile Leu Glu Arg Lys Ser Pro Gln Thr Cys Trp Gln Val Tyr Val
            340                 345                 350

Ser Asn Ser Ala Lys Tyr Ser Glu Leu Gly His Pro Phe Gly Tyr Leu
        355                 360                 365

Lys Ala Ser Thr Ala Leu Asn Cys Val Asn Leu Phe Val Met Pro Tyr
    370                 375                 380

Asn Tyr Pro Val Leu Leu Pro Leu Leu Asp Asp Leu Phe Lys Val His
385                 390                 395                 400

Lys Ala Lys Pro Thr Leu Lys Trp Arg Gln Ser Phe Glu Ser Tyr Leu
                405                 410                 415

Lys Thr Met Pro Pro Tyr Tyr Leu Gly Pro Leu Lys Lys Ala Val Arg
            420                 425                 430

Met Met Gly Ala Pro Asn Leu Ile Ala Asp Ser Met Glu Tyr Gly Leu
        435                 440                 445

Ser Tyr Ser Val Ile Ser Tyr Leu Lys Lys Leu Ser Gln Gln Ala Lys
    450                 455                 460

Ile Glu Ser Asp Arg Val Ile Gly Ser Val Gly Lys Lys Val Val Gln
465                 470                 475                 480

Glu Thr Gly Ile Lys Val Arg Ser Arg Ser His Gly Leu Ser Met Ala
                485                 490                 495

Tyr Arg Lys Asp Phe Gln Gln Leu Leu Gln Gly Ile Ser Glu Asp Val
            500                 505                 510

Pro His Arg Leu Leu Asp Leu Asn Met Lys Glu Tyr Thr Gly Phe Gln
        515                 520                 525

Val Ala Leu Leu Asn Lys Asp Leu Lys Pro Gln Thr Phe Arg Asn Ala
    530                 535                 540

Tyr Asp Ile Pro Arg Arg Asn Leu Leu Asp His Leu Thr Arg Met Arg
545                 550                 555                 560

Ser Asn Leu Leu Lys Ser Thr Arg Arg Phe Leu Lys Gly Gln Asp Glu
                565                 570                 575

Asp Gln Val His Ser Val Pro Ile Ala Gln Met Gly Asn Tyr Gln Glu
            580                 585                 590

Tyr Leu Lys Gln Val Pro Ser Pro Leu Arg Glu Leu Asp Pro Asp Gln
        595                 600                 605

Pro Arg Arg Leu His Thr Phe Gly Asn Pro Phe Lys Leu Asp Lys Lys
    610                 615                 620

Gly Met Met Ile Asp Glu Ala Asp Glu Phe Val Ala Gly Pro Gln Asn
625                 630                 635                 640

Lys His Lys Arg Pro Gly Glu Pro Asn Met Gln Gly Ile Pro Lys Arg
                645                 650                 655

Arg Arg Cys Met Ser Pro Leu Leu Arg Gly Arg Gln Gln Asn Pro Val
            660                 665                 670

Val Asn Asn His Ile Gly Gly Lys Gly Pro Pro Ala Pro Thr Thr Gln
        675                 680                 685

Ala Gln Pro Asp Leu Ile Lys Pro Leu Pro Leu His Lys Ile Ser Glu
    690                 695                 700

Thr Thr Asn Asp Ser Ile Ile His Asp Val Val Glu Asn His Val Ala
705                 710                 715                 720

Asp Gln Leu Ser Ser Asp Ile Thr Pro Asn Ala Met Asp Thr Glu Phe
                725                 730                 735

Ser Ala Ser Ser Pro Ala Ser Leu Leu Glu Arg Pro Thr Asn His Met
            740                 745                 750

Glu Ala Leu Gly His Asp His Leu Gly Thr Asn Asp Leu Thr Val Gly
        755                 760                 765

Gly Phe Leu Glu Asn His Glu Glu Pro Arg Asp Lys Glu Gln Cys Ala
    770                 775                 780

Glu Glu Asn Ile Pro Ala Ser Ser Leu Asn Lys Gly Lys Lys Leu Met
785                 790                 795                 800

His Cys Arg Ser His Glu Glu Val Asn Thr Glu Leu Lys Ala Gln Ile
                805                 810                 815

Met Lys Glu Ile Arg Lys Pro Gly Arg Lys Tyr Glu Arg Ile Phe Thr
            820                 825                 830

Leu Leu Lys His Val Gln Gly Ser Leu Gln Thr Arg Leu Ile Phe Leu
        835                 840                 845

Gln Asn Val Ile Lys Glu Ala Ser Arg Phe Lys Lys Arg Met Leu Ile
    850                 855                 860

Glu Gln Leu Glu Asn Phe Leu Asp Glu Ile His Arg Arg Ala Asn Gln
865                 870                 875                 880

Ile Asn His Ile Asn Ser Asn
                885